(19) Europäisches Patentamt / European Patent Office / Office européen des brevets

(11) **EP 4 383 098 A1**

(12) **EUROPEAN PATENT APPLICATION**
published in accordance with Art. 153(4) EPC

(43) Date of publication:
**12.06.2024 Bulletin 2024/24**

(21) Application number: **21887872.6**

(22) Date of filing: **31.12.2021**

(51) International Patent Classification (IPC):
**G06F 18/00** $^{(2023.01)}$ **G06T 7/00** $^{(2017.01)}$
**A61B 8/08** $^{(2006.01)}$

(86) International application number:
**PCT/CN2021/143710**

(87) International publication number:
**WO 2023/010797 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.08.2021 CN 202110897534**

(71) Applicant: **Wuhan Endoangel Medical Technology Co., Ltd.**
**Wuhan, Hubei 430000 (CN)**

(72) Inventors:
• **ZHENG, Biqing**
  **Wuhan, Hubei 430000 (CN)**
• **YAO, Liwen**
  **Wuhan, Hubei 430000 (CN)**
• **HU, Shan**
  **Wuhan, Hubei 430000 (CN)**
• **LIU, Qiwei**
  **Wuhan, Hubei 430000 (CN)**

(74) Representative: **Ström & Gulliksson AB**
**P.O. Box 4188**
**203 13 Malmö (SE)**

(54) **PANCREATICOBILIARY ULTRASOUND IMAGE RECOGNITION METHOD AND APPARATUS, AND SERVER**

(57) The present disclosure provides a method and device for identifying a plurality of biliopancreatic ultrasound images and a server, which determine unidentifiable biliopancreatic anatomical structures, and determine positions of the unidentifiable biliopancreatic anatomical structures in the biliopancreatic ultrasound images based on a current known positional relationship of the biliopancreatic anatomical structures, thereby identifying the biliopancreatic anatomical structures. The method comprehensively identifies and labels the biliopancreatic anatomical structures, which significantly reduces difficulty of identifying the biliopancreatic anatomical structures in the biliopancreatic ultrasound images.

obtaining the biliopancreatic ultrasound images of a biliopancreatic structure of a human body to be identified — 21

identifying and determining a plurality of biliopancreatic sites corresponding to the biliopancreatic ultrasound images of the biliopancreatic structure through a preset biliopancreatic site identification model to obtain a plurality of first biliopancreatic ultrasound images — 22

identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound image — 23

performing position identification on the third biliopancreatic ultrasound images to determine the biliopancreatic anatomical structures in the third biliopancreatic ultrasound images — 24

**FIG. 2**

## Description

FIELD OF THE INVENTION

The present disclosure relates to the technical field of medical assistance, and particularly to a method and device for identifying a plurality of biliopancreatic ultrasound images and a server.

BACKGROUND

A biliopancreatic ultrasound endoscope is an important tool for diagnosis and therapy of biliary and pancreatic diseases. A basis of endoscopic ultrasonography is that doctors use ultrasound images to accurately identify and locate biliopancreatic structures. However, biliopancreatic ultrasound images are cross-sectional images of human tissues, which mainly contain texture information that is difficult to identify with naked eyes. For endoscopists who lack professional training and long-term practice, it is difficult to accurately identify anatomical landmarks in images, which greatly affects accuracy of identifying and locating biliopancreatic structures in biliopancreatic sites.

The prior art cannot distinguish multiple biliopancreatic sites and biliopancreatic anatomical structures of a biliopancreatic structure, so it is difficult to identify the biliopancreatic structure.

SUMMARY

The present disclosure provides a method and device for identifying a plurality of biliopancreatic ultrasound images and a server to solve a problem that biliopancreatic anatomical structures of a human biliopancreatic structure cannot be better distinguished in the prior art.

The present disclosure provides a method for identifying a plurality of biliopancreatic ultrasound images, which comprises:

obtaining the biliopancreatic ultrasound images of a biliopancreatic structure of a human body to be identified;
identifying and determining a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;
identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and
performing position identification on the third biliopancreatic ultrasound images to determine the biliopancreatic anatomical structures in the third biliopancreatic ultrasound images.

In an embodiment, the obtaining the biliopancreatic ultrasound images of the biliopancreatic structure of the human body to be identified comprises:

obtaining a plurality of initial biliopancreatic ultrasound images of the biliopancreatic structure of the human body to be identified; determining an effective area in each of the initial biliopancreatic ultrasound images to obtain a plurality of the effective areas;
obtaining a horizontally circumscribed rectangle of each of the effective areas to obtain a plurality of the horizontally circumscribed rectangles; and
respectively cutting the initial biliopancreatic ultrasound images of the biliopancreatic structure to be identified along the horizontally circumscribed rectangles to obtain the biliopancreatic ultrasound images for subsequent identification.

In an embodiment, the identifying and determining the biliopancreatic sites corresponding to the biliopancreatic ultrasound images of the biliopancreatic structure through the preset biliopancreatic site identification model to obtain the first biliopancreatic ultrasound images comprises:

obtaining a plurality of preset biliopancreatic site identification initial models;
respectively training the preset biliopancreatic site identification initial models to obtain a plurality of biliopancreatic site identification models for respectively identifying different biliopancreatic sites; and
identifying and determining one biliopancreatic site corresponding to each of the first biliopancreatic ultrasound images of the biliopancreatic structure through the biliopancreatic site identification models, wherein there are multiple first biliopancreatic ultrasound images, and one biliopancreatic site corresponding to each of the biliopancreatic ultrasound images is determined.

In an embodiment, the respectively training the preset biliopancreatic site identification initial models to obtain the biliopancreatic site identification models for respectively identifying different biliopancreatic sites comprises:
training a plurality of neural network (ResNet) models to obtain the biliopancreatic site identification models, wherein the biliopancreatic site identification models respectively identify different biliopancreatic sites.

In an embodiment, the number of the preset biliopancreatic site identification initial models is eight, and the respectively training the preset biliopancreatic site identification initial models to obtain the biliopancreatic site identification models for respectively identifying different biliopancreatic sites comprises:

respectively training the eight preset biliopancreatic site identification initial models to obtain eight biliopancreatic site identification models, wherein the eight biliopancreatic site identification models are used to respectively identify an abdominal aorta site, a gastric cavity and pancreatic body site, a gastric cavity and pancreatic tail site, a confluence site, a first hepatic portal site, a gastric cavity and pancreatic head site, a duodenal bulb site, and a duodenal descending part site in the biliopancreatic ultrasound images of the biliopancreatic structure.

In an embodiment, the determining the effective area in each of the initial biliopancreatic ultrasound images to obtain the plurality of the effective areas comprises:

training an UNet++ image neural network model; and identifying the effective area in each of the initial biliopancreatic ultrasound images through the trained UNet++ image neural network model to obtain the plurality of the effective areas.

In an embodiment, the identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images comprises:

obtaining a plurality of preset biliopancreatic anatomical structure identification models; and sequentially using the preset biliopancreatic anatomical structure identification models as a target biliopancreatic anatomical structure identification model to identify and determine the biliopancreatic anatomical structures in each of the first biliopancreatic ultrasound images, wherein images in which the biliopancreatic anatomical structures are identifiable in the first biliopancreatic ultrasound images are the second biliopancreatic ultrasound images, and images in which the biliopancreatic anatomical structures are unidentifiable in the first biliopancreatic ultrasound images are the third biliopancreatic ultrasound images.

In an embodiment, the performing position identification on the third biliopancreatic ultrasound images to determine the biliopancreatic anatomical structures in the third biliopancreatic ultrasound images comprises:

determining a plurality of target regions in the third biliopancreatic ultrasound images, wherein each of the target regions is surrounded by a plurality of initial edge points; determining a coordinate origin in each of the third biliopancreatic ultrasound images to determine coordinates of the initial edge points of each of the target regions; determining coordinates of a center point of each of the target regions according to the coordinates of the initial edge points of each of the target regions; obtaining a preset positional relationship among the biliopancreatic anatomical structures; and determining the biliopancreatic anatomical structures in the target regions according to the positional relationship and the coordinates of the center point, wherein the biliopancreatic anatomical structures in the target regions are the biliopancreatic anatomical structures in the third biliopancreatic ultrasound images.

In an embodiment, the determining the coordinates of the center point of each of the target regions according to the coordinates of the initial edge points of each of the target regions comprises:

performing preset sparse and homogenization processing on the initial edge points to obtain a plurality of edge points; determining coordinates of the edge points according to the coordinate origin; and determining the coordinates of the center point of each of the target regions according to the coordinates of the edge points.

In an embodiment, the performing the preset sparse and homogenization processing on the initial edge points to obtain the edge points comprises:

traversing the initial edge points in a preset order; discarding the initial edge points whose distance between any two adjacent initial edge points is less than 10 pixels; and inserting a new initial edge point between any two adjacent initial edge points with a distance greater than 10 pixels to obtain the edge points.

In an embodiment, the determining the coordinates of the center point of each of the target regions according to the coordinates of the edge points comprises:

averaging the coordinates of the edge points of each of the target regions to obtain a mean value as the coordinates of the center point of each of the target regions.

In an embodiment, the anatomical structures are divided into seven categories, and the obtaining the preset biliopancreatic anatomical structure identification models comprises:

obtaining seven preset biliopancreatic anatomical structure identification models.

The present disclosure further provides a device for identifying a plurality of biliopancreatic ultrasound images, which comprises:

an acquisition module configured to obtain the biliopancreatic ultrasound images of a biliopancreatic structure of a human body to be identified;
a first identification module configured to identify and determine a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;
a second identification module configured to identify a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and
a positioning module configured to perform position identification on the third biliopancreatic ultrasound images to determine the biliopancreatic anatomical structures in the third biliopancreatic ultrasound images.

In an embodiment, the acquisition module is specifically configured to:

obtain a plurality of initial biliopancreatic ultrasound images of the biliopancreatic structure of the human body to be identified;
determine an effective area in each of the initial biliopancreatic ultrasound images to obtain a plurality of the effective areas;
obtain a horizontally circumscribed rectangle of each of the effective areas to obtain a plurality of the horizontally circumscribed rectangles; and
respectively cut the initial biliopancreatic ultrasound images of the biliopancreatic structure to be identified along the horizontally circumscribed rectangles to obtain the biliopancreatic ultrasound images for subsequent identification.

In an embodiment, the first identification module is specifically configured to:

obtain a plurality of preset biliopancreatic site identification initial models;
respectively train the preset biliopancreatic site identification initial models to obtain a plurality of biliopancreatic site identification models for respectively identifying different biliopancreatic sites; and
identify and determine one biliopancreatic site corresponding to each of the first biliopancreatic ultrasound images of the biliopancreatic structure through the biliopancreatic site identification models, wherein there are multiple first biliopancreatic ultrasound images, and one biliopancreatic site corresponding to each of the biliopancreatic ultrasound images is determined.

In an embodiment, the number of the preset biliopancreatic site identification initial models is eight, and the first identification module is specifically configured to: respectively train eight preset biliopancreatic site identification initial models to obtain eight biliopancreatic site identification models, wherein the eight biliopancreatic site identification models are used to respectively identify an abdominal aorta site, a gastric cavity and pancreatic body site, a gastric cavity and pancreatic tail site, a confluence site, a first hepatic portal site, a gastric cavity and pancreatic head site, a duodenal bulb site, and a duodenal descending part site in the biliopancreatic ultrasound images of the biliopancreatic structure.

In an embodiment, the second identification module is specifically configured to:

obtain a plurality of preset biliopancreatic anatomical structure identification models; and
sequentially use the preset biliopancreatic anatomical structure identification models as a target biliopancreatic anatomical structure identification model to identify and determine the biliopancreatic anatomical structures in each of the first biliopancreatic ultrasound images, wherein images in which the biliopancreatic anatomical structures are identifiable in the first biliopancreatic ultrasound images are the second biliopancreatic ultrasound images, and images in which the biliopancreatic anatomical structures are unidentifiable in the first biliopancreatic ultrasound images are the third biliopancreatic ultrasound images.

In an embodiment, the positioning module is specifically configured to:

determine a plurality of target regions in the third biliopancreatic ultrasound images, wherein each of the target regions is surrounded by a plurality of initial edge points;
determine a coordinate origin in each of the third biliopancreatic ultrasound images to determine coordinates of the initial edge points of each of the target regions;

determine coordinates of a center point of each of the target regions according to the coordinates of the initial edge points of each of the target regions;

obtain a preset positional relationship of the biliopancreatic anatomical structures; and

determine the biliopancreatic anatomical structures in the target regions according to the positional relationship and the coordinates of the center point, wherein the biliopancreatic anatomical structures in the target regions are the biliopancreatic anatomical structures in the third biliopancreatic ultrasound images.

In an embodiment, the positioning module is specifically configured to:

perform preset sparse and homogenization processing on the initial edge points to obtain a plurality of edge points;

determine coordinates of the edge points according to the coordinate origin; and

determine the coordinates of the center point of each of the target regions according to the coordinates of the edge points.

The present disclosure further provides a server comprising one or more processors, a memory, and one or more application programs. The application programs are stored in the memory and are configured to be executed by the processors to implement the aforementioned method for identifying the biliopancreatic ultrasound images.

The present disclosure further provides a computer-readable storage medium storing a computer program. The computer program is loaded by a processor to perform the aforementioned method for identifying the biliopancreatic ultrasound images.

The present disclosure provides a method and device for identifying a plurality of biliopancreatic ultrasound images and a server, which determine unidentifiable biliopancreatic anatomical structures through current biliopancreatic site identification and biliopancreatic anatomical structure identification, and determine positions of the unidentifiable biliopancreatic anatomical structures in the biliopancreatic ultrasound images based on a current known positional relationship of the biliopancreatic anatomical structures, thereby identifying the biliopancreatic anatomical structures. The method combines current biliopancreatic site information, image features of the biliopancreatic anatomical structures, and position coordinates of the biliopancreatic anatomical structures to comprehensively identify and label the biliopancreatic anatomical structures, which significantly reduces difficulty of identifying the biliopancreatic anatomical structures in the biliopancreatic ultrasound images.

BRIEF DESCRIPTION OF DRAWINGS

In order to more clearly illustrate technical solutions in embodiments of the present disclosure, a brief description of accompanying drawings used in the embodiments will be given below. The accompanying drawings in the following description are merely some embodiments of the present disclosure. For those skilled in the art, other drawings may be obtained from these accompanying drawings without creative labor.

FIG. 1 is a scenario schematic diagram of lesion identification according to an embodiment of the present disclosure.

FIG. 2 is a schematic flowchart of a method for identifying a plurality of biliopancreatic ultrasound images according to an embodiment of the present disclosure.

FIG. 3 is a schematic diagram of standard eight sites and biliopancreatic anatomical structures thereof according to an embodiment of the present disclosure.

FIG. 4 is a schematic diagram of the biliopancreatic anatomical structures according to an embodiment of the present disclosure.

FIG. 5 is a schematic diagram of identifiable biliopancreatic anatomical structures according to an embodiment of the present disclosure.

FIG. 6 is a schematic diagram of unidentifiable biliopancreatic anatomical structures according to an embodiment of the present disclosure.

FIG. 7 is a schematic flowchart of obtaining the biliopancreatic ultrasound images according to an embodiment of the present disclosure.

FIG. 8 is a schematic flowchart of identifying the biliopancreatic sites according to an embodiment of the present disclosure.

FIG. 9 is a schematic flowchart of performing position identification according to an embodiment of the present disclosure.

FIG. 10 is a schematic diagram of identification situations of different biliopancreatic anatomical structure identification models according to an embodiment of the present disclosure.

FIG. 11 is a schematic diagram of a positional relationship according to an embodiment of the present disclosure.

FIG. 12 is a schematic diagram of a device for identifying the biliopancreatic ultrasound images according to an embodiment of the present disclosure.

FIG. 13 is a schematic structural diagram of a server according to an embodiment of the present disclosure.

DETAILED DESCRIPTION OF EMBODIMENTS

Technical solutions in embodiments of the present disclosure will be clearly and completely described below in conjunction with accompanying drawings in the embodiments of the present disclosure. It is apparent that the described embodiments are merely a part of the embodiments of the present disclosure and not all embodiments. All other embodiments obtained by those skilled in the art based on the embodiments of the present disclosure without creative labor are within the claimed scope of the present disclosure.

In the description of the present disclosure, it should be understood that location or position relationships indicated by terms, such as "center", "longitudinal", "transverse", "length", "width", "thickness", "up", "down", "front", "rear", "left", "right", "vertical", "horizontal", "top", "bottom", "within", "outside", "clockwise", and "counterclockwise", are location or position relationships based on illustration of the accompanying drawings, are merely used for describing the present disclosure and simplifying the description instead of indicating or implying the indicated apparatuses or elements should have specified locations or be constructed and operated according to specified locations, and Thereof, should not be intercepted as limitations to the present disclosure. Furthermore, terms such as "first" and "second" are used merely for description, but shall not be construed as indicating or implying relative importance or implicitly indicating a number of the indicated technical feature. Hence, the feature defined with "first" and "second" may explicitly or implicitly includes one or more such features. In the description of the present disclosure, a term "a plurality of" means "two or more" unless otherwise specifically limited.

In the present disclosure, a term "exemplary" means "serving as an example, instance, or illustration." Any embodiment described herein as "exemplary" is not necessarily construed as being more preferable or advantageous than other embodiments. The following description is presented to enable any skilled in the art to make and use the present invention. Details are set forth in the following description for purpose of explanation. It should be noted that those skilled in the art can realize that the present invention can also be implemented without using these specific details. In other embodiments, well-known structures and processes are not elaborated, so as to prevent unnecessary details from obscuring a description of the present invention. Therefore, the present invention is not intended to be limited by the embodiments, but is accorded with the widest scope consistent with principles and features disclosed herein.

The present disclosure provides a method and device for identifying a plurality of biliopancreatic ultrasound images and a server, which will be described in detail below.

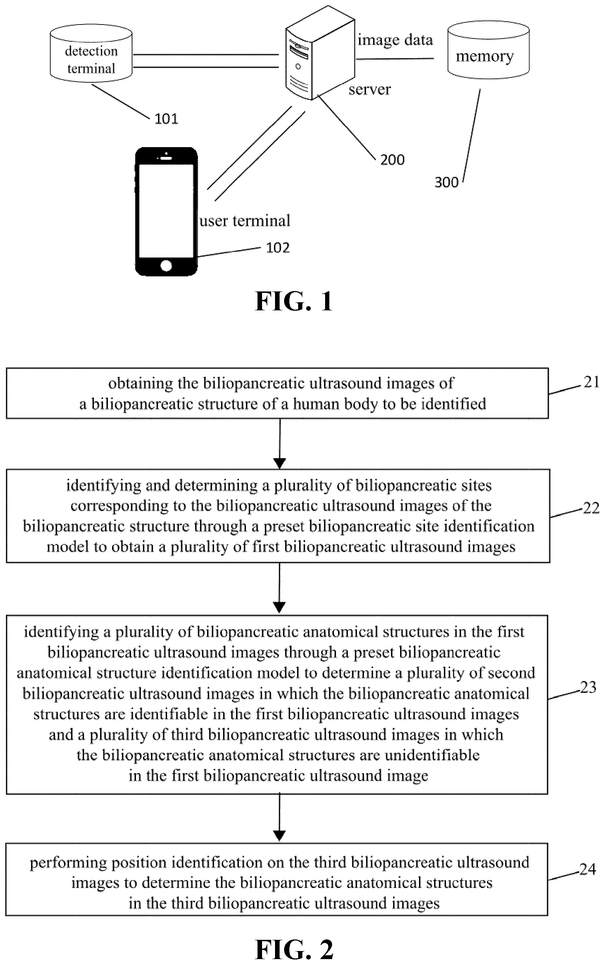

Please refer to FIG. 1, which is a scenario schematic diagram of a biliopancreatic ultrasound image identification system according to an embodiment of the present disclosure. The biliopancreatic ultrasound image identification system comprises a plurality of terminals 100 and a server 200. The terminals 100 and the server 200 are connected and communicated with each other through Internet composed of various gateways, which will not be described in detail herein. The terminals 100 may comprise a detection terminal 101, a user terminal 102, and the like.

In this embodiment of the present disclosure, the server 200 is mainly configured to obtain a plurality of biliopancreatic ultrasound images of a biliopancreatic structure of a human body to be identified; identify and determine a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined; identify a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and perform position identification on the third biliopancreatic ultrasound images to determine the biliopancreatic anatomical structures in the third biliopancreatic ultrasound images.

In this embodiment of the present disclosure, the server 200 may be an independent server, or may be a server network or server cluster composed of multiple servers. For example, the server 200 described in this embodiment of the present disclosure includes, but is not limited to, a computer, a network host, a single network server, a set of multiple network servers, or a cloud server composed of multiple servers. The cloud server is composed of a large number of computers or network servers based on cloud computing. In this embodiment of the present disclosure, the server and the terminals may be communicated with each other through any communication method comprising, but not limited to, mobile communication based on 3rd generation partnership project (3GPP), long term evolution (LTE), or worldwide interoperability for microwave access (WiMAX), or computer network communication based on TCP/IP protocol suite (TCP/IP) or user datagram protocol (UDP).

It can be understood that the terminals 100 used in this embodiment of the present disclosure may be a device comprising both receiving and transmitting hardware, that is, a device having receiving and transmitting hardware capable of performing bidirectional communication on a bidirectional communication link. Each of the terminals may comprise a cellular or other communication device with a single-line display device or a multi-line display device, or without a multi-line display device.

Specifically, the detection terminal 101 is mainly configured to collect endoscopic images of a to-be-detected part of the human body. Acquisition equipment of the detection terminal may be electronic equipment such as a magnetic resonance imaging (MRI) apparatus, a computed tomography (CT) apparatus, a colposcope, or an endoscope. In this embodiment, the image acquisition equipment may be a biliopancreatic ultrasound endoscope, which is mainly configured to acquire biliopancreatic ultrasound images of a human biliopancreatic structure.

The user terminal 102 may be, but is not limited to, a portable terminal such as a mobile phone or a tablet computer, a fixed terminal such as a computer and a query machine, or a virtual terminal. The user terminal 102 is mainly configured to upload and process the biliopancreatic ultrasound images, and to display processing results corresponding to the images.

It can be understood by those skilled in the art that an application environment shown in FIG. 1 is only a kind of application scenario of the present disclosure, and does not constitute a limitation to the application scenario of the present disclosure. Other application environments may comprise more or fewer servers than the server shown in FIG. 1, or other network connection relationships of the servers. For example, FIG. 1 shows only one server and two terminals. It can be understood that the scenario of lesion identification may further comprise one or more other servers, or/and one or more terminals connected to the servers through a network, which will not be described in detail herein.

In addition, as shown in FIG. 1, the biliopancreatic ultrasound image identification system may further comprise a memory 300 for storing data, such as storing image data, for example, image data of the to-be-detected part acquired by the terminal. The memory 300 may comprise a local database and/or a cloud database.

It should be noted that the scenario schematic diagram of the biliopancreatic ultrasound image identification system shown in FIG. 1 is only an example. Lesion identification scenarios and scenarios described in embodiments of the present disclosure are for a purpose of illustrating technical solutions of the embodiments of the present disclosure more clearly, and do not constitute a limitation on the technical solutions of the embodiments of the present disclosure. Those skilled in the art know that with evolution of biliopancreatic ultrasound image identification scenarios and emergence of new business scenarios, the technical solutions of the embodiments of the present disclosure are also applicable to similar technical problems.

Please refer to FIG. 2, which is a schematic flowchart of a method for identifying a plurality of biliopancreatic ultrasound images according to an embodiment of the present disclosure. The method may comprise the following steps.

Step 21: obtaining the biliopancreatic ultrasound images of a biliopancreatic structure of a human body to be identified.

The method for identifying the biliopancreatic ultrasound images provided by the embodiment of the present disclosure is mainly used to identify the biliopancreatic structure of the human body, so that a doctor can determine pathological changes in the biliopancreatic structure according to the biliopancreatic ultrasound images corresponding to the biliopancreatic structure.

Specifically, the biliopancreatic ultrasound images corresponding to the biliopancreatic structure may be directly obtained by the biliopancreatic ultrasound endoscope.

Step 22: identifying and determining a plurality of biliopancreatic sites corresponding to the biliopancreatic ultrasound images of the biliopancreatic structure through a preset biliopancreatic site identification model to obtain a plurality of first biliopancreatic ultrasound images.

For identification of the biliopancreatic structure in the embodiment of the present disclosure, a standard scan of the biliopancreatic ultrasound endoscope is divided into a scan of multiple biliopancreatic sites and a scan of multiple biliopancreatic anatomical structures. The doctor needs to complete the scan of all biliopancreatic sites and identification of all biliopancreatic anatomical structures to ensure a comprehensive view of a biliopancreatic system.

Therefore, in the embodiment of the present disclosure, the biliopancreatic ultrasound images of the biliopancreatic structure may be identified by using the preset biliopancreatic site identification model to determine the biliopancreatic sites corresponding to the biliopancreatic ultrasound images, and each of the biliopancreatic ultrasound images corresponds to only one biliopancreatic site.

After identifying the biliopancreatic sites corresponding to the biliopancreatic ultrasound images, the first biliopancreatic ultrasound images are obtained, and one biliopancreatic site corresponding to each of the first biliopancreatic ultrasound images is determined.

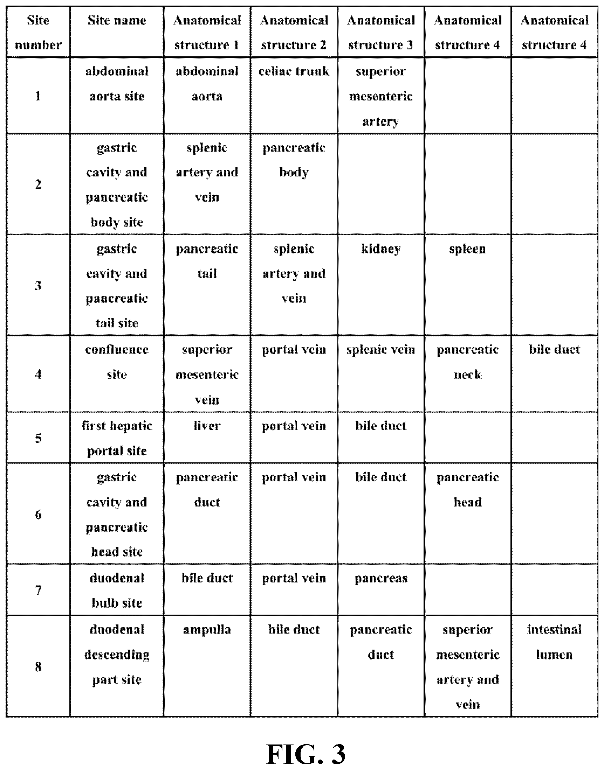

In a specific embodiment of the present disclosure, the standard scan of the biliopancreatic ultrasound endoscope has eight sites, and each of the sites has corresponding biliopancreatic anatomical structures. Please refer to FIG. 3, which is a schematic diagram of standard eight sites and biliopancreatic anatomical structures thereof according to an embodiment of the present disclosure.

In FIG. 3, taking an abdominal aorta site as an example, the abdominal aorta site corresponds to three biliopancreatic anatomical structures, namely: an abdominal aorta, a celiac trunk, and a superior mesenteric artery. Taking a gastric cavity and pancreatic body site as an example, the gastric cavity and pancreatic body site comprises two biliopancreatic anatomical structures, i.e., a splenic artery and vein, and a pancreatic body. In the embodiments of the present disclosure, each of the sites corresponds to different biliopancreatic anatomical structures.

Step 23: identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images.

After identifying the biliopancreatic site corresponding to each of the first biliopancreatic ultrasound images, it is necessary to further identify the plurality of biliopancreatic anatomical structures in each of the first biliopancreatic ultrasound images. The biliopancreatic anatomical structures in the first biliopancreatic ultrasound images obtained by identifying the biliopancreatic site may be identified by using the preset biliopancreatic anatomical structure identification model.

It should be noted that in the prior art for identifying biliopancreatic anatomical structures, the biliopancreatic anatomical structures are identified on biliopancreatic ultrasound images whose biliopancreatic sites have been identified. However, in a process of identifying the biliopancreatic anatomical structures, because textures of some of the biliopancreatic anatomical structures are substantially same, and some of the biliopancreatic anatomical structures are anechoic structures, it is not possible to identify all of the biliopancreatic anatomical structures.

Therefore, the preset biliopancreatic anatomical structure identification model can only identify some of the biliopancreatic anatomical structures. That is, the preset biliopancreatic anatomical structure identification model can determine the plurality of second biliopancreatic ultrasound images in which the biliopancreatic anatomical structures are identifiable in the first biliopancreatic ultrasound images and the plurality of third biliopancreatic ultrasound images in which the biliopancreatic anatomical structures are unidentifiable in the first biliopancreatic ultrasound images.

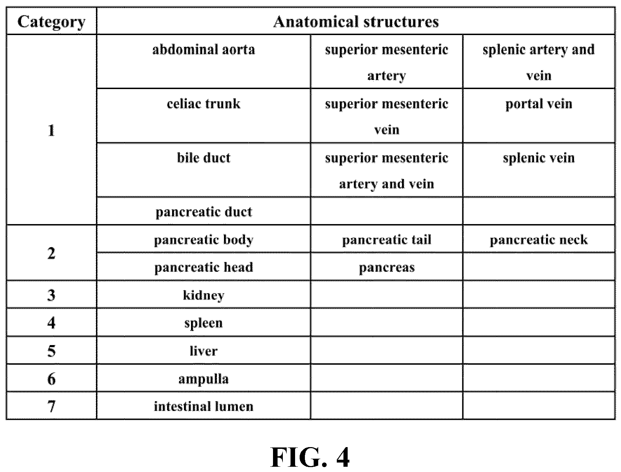

Please refer to FIG. 4, which is a schematic diagram of the biliopancreatic anatomical structures according to an embodiment of the present disclosure. In FIG. 4, instead of identifying the biliopancreatic sites of the biliopancreatic ultrasound images of the biliopancreatic structure, the biliopancreatic anatomical structures are first divided into seven categories. Then, the biliopancreatic anatomical structures in the biliopancreatic ultrasound images of the biliopancreatic structure are identified according to the seven categories of the biliopancreatic anatomical structures, so as to classify the biliopancreatic structure according to the biliopancreatic anatomical structures.

It should be noted that in the biliopancreatic sites shown in FIG. 3, different biliopancreatic sites may comprise one or more same biliopancreatic anatomical structures. Therefore, when the biliopancreatic anatomical structures are classified in FIG. 4, the number of the biliopancreatic anatomical structures included in FIG. 4 is less than the number of the biliopancreatic anatomical structures corresponding to all biliopancreatic sites in FIG. 3.

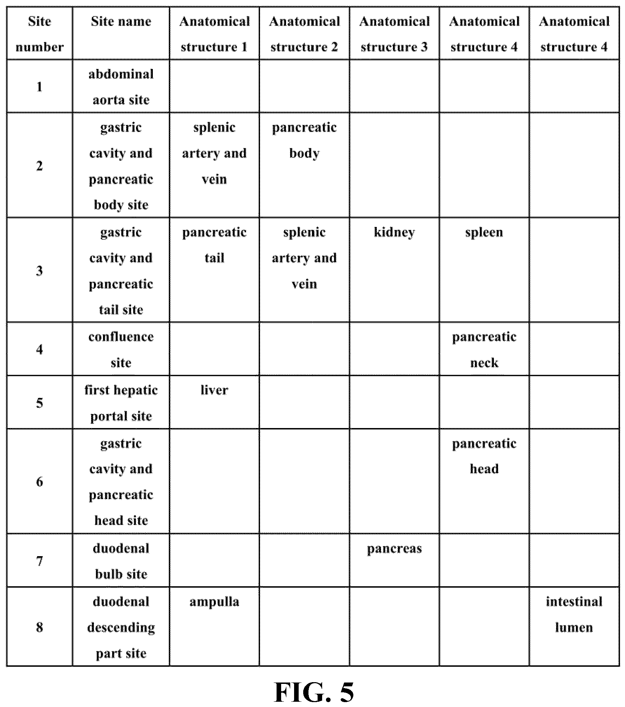

Please refer to FIG. 5, which is a schematic diagram of identifiable biliopancreatic anatomical structures according to an embodiment of the present disclosure. In FIG. 5, the aforementioned biliopancreatic site identification and biliopancreatic anatomical structure identification can effectively identify different biliopancreatic anatomical structures corresponding to different biliopancreatic sites, but not all biliopancreatic anatomical structures can be identified.

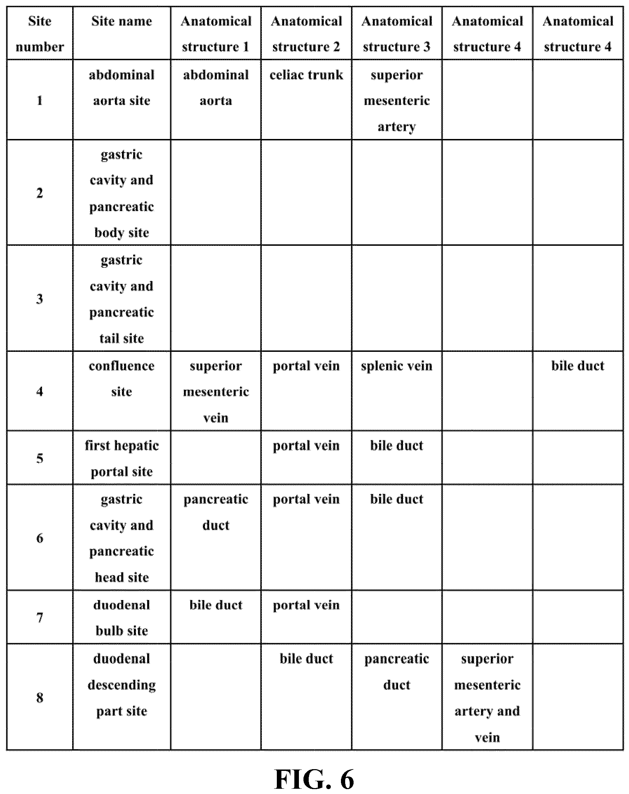

Take a first hepatic portal site as an example. Normally, the first hepatic portal site corresponds to three biliopancreatic anatomical structures: a liver, a portal vein, and a bile duct. The aforementioned biliopancreatic site identification and biliopancreatic anatomical structure identification can only identify the biliopancreatic anatomical structure corresponding to the liver, but cannot identify the portal vein and the bile duct. FIG. 5 shows the identifiable structures. FIG. 6 shows the unidentifiable biliopancreatic anatomical structures.

In the embodiment of the present disclosure, in the first biliopancreatic ultrasound images, images in which the biliopancreatic anatomical structures are identifiable are the second biliopancreatic ultrasound images, and images in which the biliopancreatic anatomical structures are unidentifiable are the third biliopancreatic ultrasound images. Then, it is only necessary to identify and determine the biliopancreatic anatomical structures in the third biliopancreatic ultrasound images.

It should be noted that in the above embodiment, when the preset biliopancreatic anatomical structure identification model is used for identification, the preset biliopancreatic anatomical structure identification model identifies the first biliopancreatic ultrasound images whose biliopancreatic sites has been determined.

Step 24: performing position identification on the third biliopancreatic ultrasound images to determine the biliopancreatic anatomical structures in the third biliopancreatic ultrasound images.

After the third biliopancreatic ultrasound images corresponding to the unidentifiable biliopancreatic anatomical structures are determined, the position identification may be performed on the third biliopancreatic ultrasound images to determine the unidentifiable biliopancreatic anatomical structures in the third biliopancreatic ultrasound images.

The method for identifying the biliopancreatic ultrasound images provided by the present disclosure determines unidentifiable biliopancreatic anatomical structures through current biliopancreatic site identification and biliopancreatic anatomical structure identification, and determines positions of the unidentifiable biliopancreatic anatomical structures in the biliopancreatic ultrasound images based on a current known positional relationship of the biliopancreatic anatomical structures, thereby identifying the biliopancreatic anatomical structures. The method combines current biliopancreatic site information, image features of the biliopancreatic anatomical structures, and position coordinates of the biliopancreatic anatomical structures to comprehensively identify and label the biliopancreatic anatomical structures, which significantly reduces difficulty of identifying the biliopancreatic anatomical structures in the biliopancreatic ultrasound images.

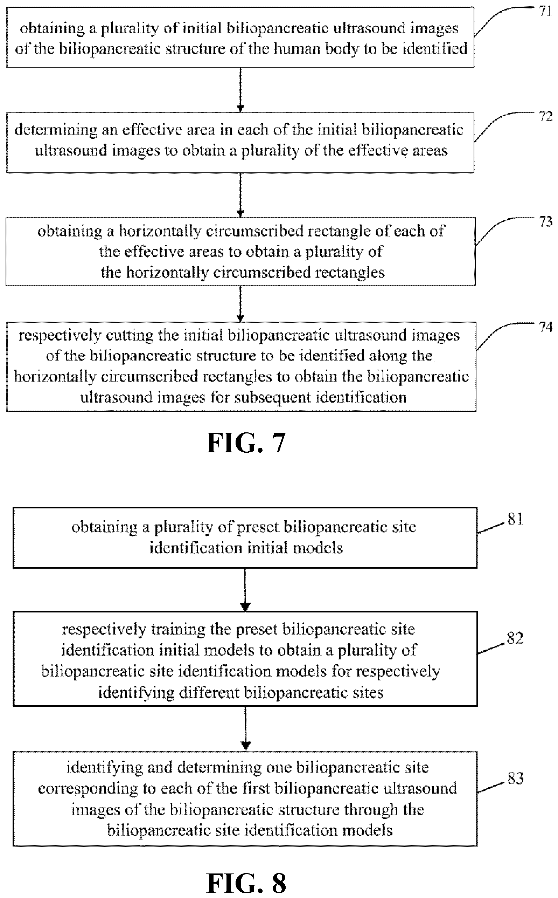

In an embodiment of the present disclosure, a plurality of initial biliopancreatic ultrasound images corresponding to the biliopancreatic structure are obtained by the biliopancreatic ultrasound endoscope, and then the initial biliopancreatic ultrasound images need to be processed to obtain the biliopancreatic ultrasound images that can be subsequently identified. Please refer to FIG. 7, which is a schematic flowchart of obtaining the biliopancreatic ultrasound images according to an embodiment of the present disclosure, whichmay comprise the following steps.

Step 71: obtaining a plurality of initial biliopancreatic ultrasound images of the biliopancreatic structure of the human body to be identified.

Step 72: determining an effective area in each of the initial biliopancreatic ultrasound images to obtain a plurality of the effective areas.

Step 73: obtaining a horizontally circumscribed rectangle of each of the effective areas to obtain a plurality of the horizontally circumscribed rectangles.

Step 74: respectively cutting the initial biliopancreatic ultrasound images of the biliopancreatic structure to be identified along the horizontally circumscribed rectangles to obtain the biliopancreatic ultrasound images for subsequent identification.

Specifically, the initial biliopancreatic ultrasound images of the biliopancreatic structure obtained by the biliopancreatic ultrasound endoscope comprise a lot of redundant information. The redundant information will have an impact on the subsequent identification of biliopancreatic sites and biliopancreatic anatomical structures, so the redundant information needs to be removed. Specifically, the effective areas in the initial biliopancreatic ultrasound images may be sequentially determined, and other areas other than the effective areas are cropped and removed.

In a specific embodiment, after the effective area in each of the initial biliopancreatic ultrasound images is determined, the horizontally circumscribed rectangle corresponding to the effective area can be directly determined. The effective area is retained according to a shape of the horizontally circumscribed rectangle, and the other areas outside the horizontally circumscribed rectangle are cut out to obtain the biliopancreatic ultrasound image of the biliopancreatic structure.

In an embodiment of the present disclosure, a neural network model may be used to determine the effective areas in the initial biliopancreatic ultrasound images. Specifically, an UNet++ image neural network model may be trained to identify the effective areas in the initial biliopancreatic ultrasound images, and to crop the initial biliopancreatic ultrasound images. For a specific training process, reference may be made to the prior art, which is not limited herein.

It should be noted that in the embodiment of the present disclosure, there are multiple initial biliopancreatic ultrasound images, and the effective areas of the initial biliopancreatic ultrasound images are different. Therefore, the horizontally circumscribed rectangles corresponding to the effective areas of the initial biliopancreatic ultrasound images are also different, and the biliopancreatic ultrasound images obtained by cropping are also different.

Please refer to FIG. 8, which is a schematic flowchart of identifying the biliopancreatic sites according to an embodiment of the present disclosure, which may comprise the following steps.

Step 81: obtaining a plurality of preset biliopancreatic site identification initial models.

Step 82: respectively training the preset biliopancreatic site identification initial models to obtain a plurality of biliopancreatic site identification models for respectively identifying different biliopancreatic sites.

Step 83: identifying and determining one biliopancreatic site corresponding to each of the first biliopancreatic ultrasound images of the biliopancreatic structure through the biliopancreatic site identification models.

In an embodiment of the present disclosure, a neural network model may be used to identify different biliopancreatic sites corresponding to the biliopancreatic ultrasound images. Specifically, multiple neural network (ResNet) models may be trained to obtain the biliopancreatic site identification models, and the biliopancreatic site identification models respectively identify different biliopancreatic sites.

In some embodiments of the present disclosure, one neural network (ResNet) model may be trained to identify different biliopancreatic sites at the same time.

In a specific embodiment, the number of the preset biliopancreatic site identification initial models is eight. The eight preset biliopancreatic site identification initial models are respectively trained to obtain eight biliopancreatic site identification models. The eight biliopancreatic site identification models are used to respectively identify an abdominal aorta site, a gastric cavity and pancreatic body site, a gastric cavity and pancreatic tail site, a confluence site, a first hepatic portal site, a gastric cavity and pancreatic head site, a duodenal bulb site, and a duodenal descending part site in the biliopancreatic ultrasound images of the biliopancreatic structure.

After identifying the biliopancreatic sites in the biliopancreatic ultrasound images to obtain the first biliopancreatic ultrasound images, the identification of the biliopancreatic anatomical structures is performed. Specifically, a plurality of preset biliopancreatic anatomical structure identification initial models may be obtained, and the preset biliopancreatic anatomical structure identification initial models may be trained to obtain a plurality of biliopancreatic anatomical structure identification models.

After the preset biliopancreatic anatomical structure identification models are obtained, the preset biliopancreatic anatomical structure identification models are sequentially used as a target biliopancreatic anatomical structure identification model to identify and determine the biliopancreatic anatomical structures in each of the first biliopancreatic ultrasound images.

Specifically, one biliopancreatic anatomical structure identification model can only identify one category of the biliopancreatic anatomical structures. Therefore, for one first biliopancreatic ultrasound image, it is necessary to use all the biliopancreatic anatomical structure identification models to identify the biliopancreatic anatomical structures in the first biliopancreatic ultrasound image, so as to ensure that all biliopancreatic anatomical structures in the first biliopancreatic ultrasound image are identified.

In a specific embodiment, as shown in FIG. 4, the biliopancreatic anatomical structures are divided into seven categories. Therefore, the number of the biliopancreatic anatomical structure identification initial models may be seven. Specifically, the biliopancreatic anatomical structure identification initial models may be UNet++ neural network models. Seven UNet++ neural network models are trained to identify different categories of the biliopancreatic anatomical structures.

Because there are some unidentifiable biliopancreatic anatomical structures, as shown in FIG. 5, it is necessary to identify and determine the unidentifiable biliopancreatic anatomical structures. The images in which the biliopancreatic anatomical structures are identifiable in the first biliopancreatic ultrasound images are the second biliopancreatic ultrasound images. The images in which the biliopancreatic anatomical structures are unidentifiable in the first biliopancreatic ultrasound images are the third biliopancreatic ultrasound images. Accordingly, it is necessary to identify the biliopancreatic anatomical structures in the third biliopancreatic ultrasound images.

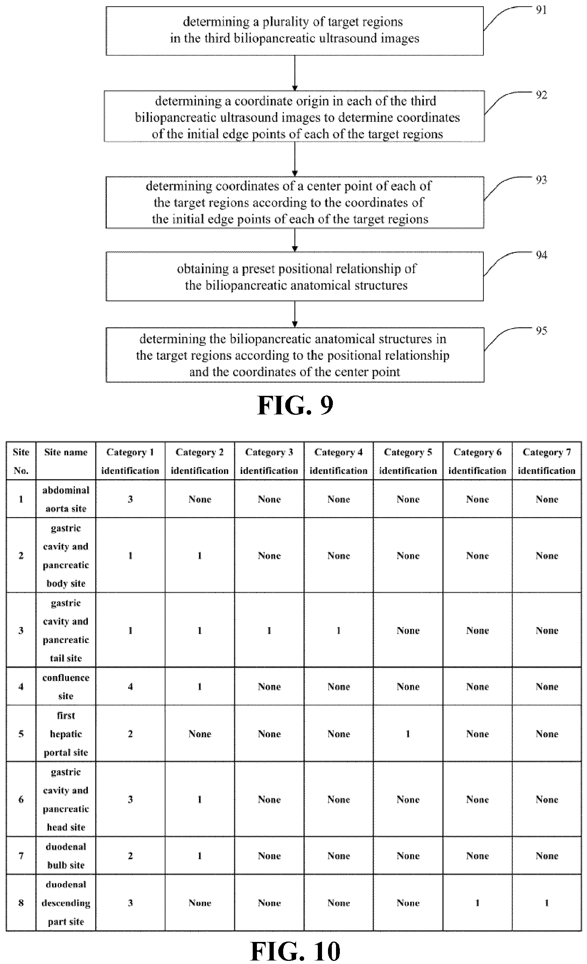

Please refer to FIG. 9, which is a schematic flowchart of performing position identification according to an embodiment of the present disclosure, which may comprise the following steps.

Step 91: determining a plurality of target regions in the third biliopancreatic ultrasound images.

Please refer to FIG. 10, which is a schematic diagram of identification situations of different biliopancreatic anatomical structure identification models according to an embodiment of the present disclosure. In some embodiments of the present disclosure, the biliopancreatic anatomical structures of each of the biliopancreatic sites are known. Therefore, if the biliopancreatic ultrasound image of the biliopancreatic structure does not comprise one certain biliopancreatic anatomical structure, the biliopancreatic anatomical structure identification model will not perform corresponding identification of the biliopancreatic anatomical structure on the biliopancreatic ultrasound image.

That is, if one certain biliopancreatic anatomical structure does not exist in the biliopancreatic site, the biliopancreatic anatomical structure identification model that can identify the biliopancreatic anatomical structure will not perform identification on the first biliopancreatic ultrasound image, which saves time for the identification of the biliopancreatic anatomical structures and improves efficiency of the identification.

Specifically, please refer to FIG. 4 and FIG. 10. As shown in FIG. 4, the abdominal aorta, the celiac trunk, and the superior mesenteric artery belong to one same category of the biliopancreatic anatomical structures. Therefore, the three biliopancreatic anatomical structures may be identified by one same biliopancreatic anatomical structure identification model. That is, three target regions in the third biliopancreatic ultrasound image corresponding to the abdominal aorta may be determined by a category 1 identification model.

For the first hepatic portal site, the biliopancreatic anatomical structures corresponding to the first hepatic portal site comprise the liver, the portal vein, and the bile duct. The portal vein and the bile duct belong to one same category of the biliopancreatic anatomical structures. The liver belongs to another category of the biliopancreatic anatomical structures. Therefore, when the biliopancreatic anatomical structure identification model is used for recognition, it is not only necessary to use the category 1 identification model to identify two target regions, but also to use a category 5 identification model to identify one target region, for a total of three target regions. The identified three target regions respectively correspond to the liver, the portal vein, and the bile duct.

Step 92: determining a coordinate origin in each of the third biliopancreatic ultrasound images to determine coordinates of the initial edge points of each of the target regions.

In an embodiment of the present disclosure, an upper left vertex of the third biliopancreatic ultrasound image may be used as a coordinate origin to construct a coordinate system. Each of the target regions is surrounded by a plurality of initial edge points (x, y).

Step 93: determining coordinates of a center point of each of the target regions according to the coordinates of the initial edge points of each of the target regions.

After determining the initial edge points of each of the target regions, the number of the initial edge points is relatively large, and the initial edge points are not necessarily evenly distributed. In order to ensure accuracy of calculation of the coordinates of the center point of each of the target regions and simplify the calculation, it is necessary to perform preset sparse and homogenization processing on the initial edge points to obtain a plurality of edge points, determine coordinates of the edge points according to the coordinate origin, and determine the coordinates of the center point of each of the target regions according to the coordinates of the edge points.

Specifically, the performing the preset sparse and homogenization processing on the initial edge points may comprises: traversing the initial edge points in a preset order, discarding the initial edge points whose distance between any two adjacent initial edge points is less than 10 pixels, and inserting a new initial edge point between any two adjacent initial edge points with a distance greater than 10 pixels to obtain the edge points.

Furthermore, a coordinate sequence $\{(xl, yl), (x2, y2)...(xn, yn)\}$ corresponding to the edge points can be obtained according to the coordinate origin. In an embodiment of the present disclosure, a mean value of the coordinates of all edge points may be used as the coordinates of the center point of the target region. That is, the coordinates Rc (Xc, Yc) of the center point of the target region may be:

$$Xc = (x1+x2+......+xn) / n$$

$$Yc = (y1+y2+......+yn) / n$$

It should be noted that the calculation of the coordinates of the center point is for one certain target region, and the coordinates of the center points of different target regions are different.

Step 94: obtaining a preset positional relationship of the biliopancreatic anatomical structures.

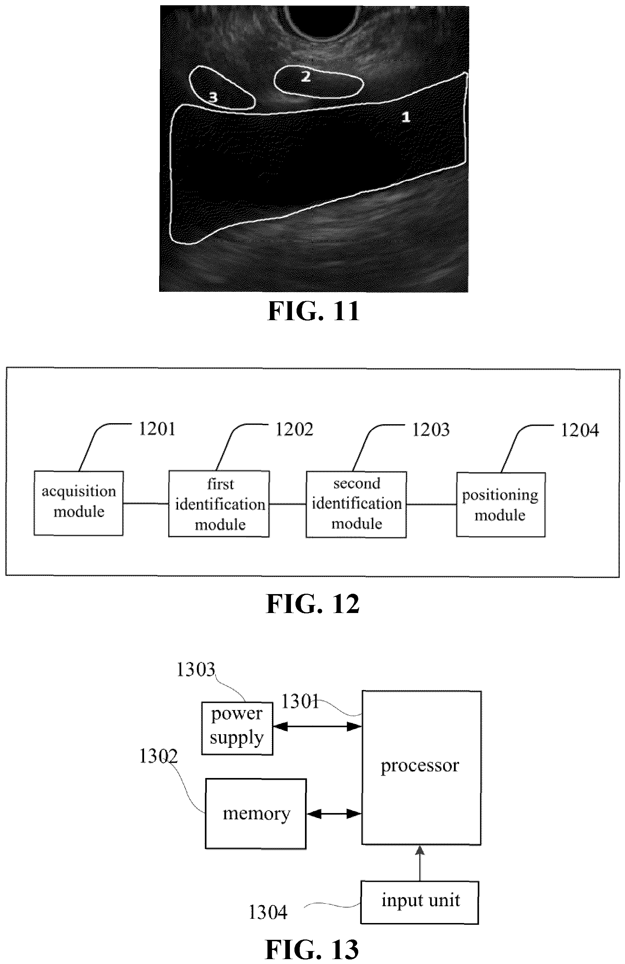

In an embodiment of the present disclosure, a known positional relationship among multiple biliopancreatic anatomical structures may be acquired. Please refer to FIG. 11, which is a schematic diagram of a positional relationship according to an embodiment of the present disclosure. FIG. 11 shows a positional relationship among the abdominal aorta 1, the celiac trunk 2, and the superior mesenteric artery 3.

Step 95: determining the biliopancreatic anatomical structures in the target regions according to the positional relationship and the coordinates of the center point.

In the embodiment of the present disclosure, please refer to FIG. 4 and FIG. 11, according to FIG. 4, it can be determined that the category 1 identification model can identify the three target regions in the abdominal aorta site. The positional relationship among the three biliopancreatic anatomical structures, i.e., the abdominal aorta, the celiac trunk, and the superior mesenteric artery, in the abdominal aortic site is known and determined. Therefore, it is only necessary to match the coordinates of the center points of the three target regions with the known positional relationship, and then the biliopancreatic anatomical structures corresponding to the three target regions can be determined.

Specifically, the three target regions of the abdominal aorta site are Rc1, Rc2, and Rc3. The three target regions correspond to the coordinates of the three center points, respectively. According to the positional relationship, it can be determined that in the coordinates of the three center points, the target region with the largest Xc and Yc is the abdominal aorta, the target region with the smallest Xc is the superior mesenteric artery, and the other is the celiac trunk.

In an embodiment of the present disclosure, the coordinates of the center point of the target region may be used as the position coordinates of the target region. Therefore, a position of one biliopancreatic anatomical structure may be determined by judging the preset positional relationship and the coordinates of the center point, so that the biliopancreatic anatomical structure corresponding to the target region is determined. The biliopancreatic anatomical structures corresponding to the target regions are determined sequentially by the above method.

It should be noted that the term "None" in FIG. 10 represents that one corresponding first biliopancreatic ultrasound image does not need to be identified by one corresponding category identification model (i.e. the biliopancreatic anatomical structure identification model). The term "1" in FIG. 10 represents that only one target region can be identified by one corresponding category identification model. Furthermore, because the biliopancreatic anatomical structures that can be identified by each of the category identification models are determined, when the only one target region is identified, the biliopancreatic anatomical structure corresponding to the target region has been determined. Therefore, there is no need to use the positional relationship for subsequent identification.

Taking the gastric cavity and pancreatic body site as an example, the category 1 identification model and a category 2 identification model respectively identify the target regions in the gastric cavity and pancreatic body site to obtain two target regions. Among the biliopancreatic anatomical structures that can be identified by the category 1 identification model, only the splenic artery and vein belong to the gastric cavity and pancreatic body site. Therefore, when the category 1 identification model identifies and obtain one target region, it can be directly confirmed that the target region identified by the category 1 identification model corresponds to the splenic artery and vein. Similarly, among the biliopancreatic anatomical structures that can be identified by the category 2 identification model, only the pancreatic body belongs to the gastric cavity and pancreatic body site. Therefore, when the category 2 identification model identifies and obtain one target region, it can be directly confirmed that the target region identified by the category 2 identification model corresponds to the pancreatic body.

Furthermore, in FIG. 10, if one same category identification model identifies multiple target regions, it is necessary to determine the biliopancreatic anatomical structure corresponding to each of the multiple target regions according to the positional relationship of the multiple biliopancreatic anatomical structures.

It should be noted that in the embodiments of the present disclosure, a process of training the neural network may refer to the prior art, which is not limited herein.

In order to better implement the method for identifying the biliopancreatic ultrasound images provided by the disclosure, on a basis of the method for identifying the biliopancreatic ultrasound images, the disclosure further provides a device for identifying the biliopancreatic ultrasound images. Please refer to FIG. 12, which is a schematic diagram of a device for identifying the biliopancreatic ultrasound images according to an embodiment of the present disclosure. The device for identifying the biliopancreatic ultrasound images comprises:

an acquisition module 1201 configured to obtain the biliopancreatic ultrasound images of a biliopancreatic structure of a human body to be identified;
a first identification module 1202 configured to identify and determine a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;
a second identification module 1203 configured to identify a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and
a positioning module 1204 configured to perform position identification on the third biliopancreatic ultrasound images to determine the biliopancreatic anatomical structures in the third biliopancreatic ultrasound images.

The device for identifying the biliopancreatic ultrasound images provided by the present disclosure determines unidentifiable biliopancreatic anatomical structures through current biliopancreatic site identification and biliopancreatic anatomical structure identification, and determines positions of the unidentifiable biliopancreatic anatomical structures in the biliopancreatic ultrasound images based on a current known positional relationship of the biliopancreatic anatomical structures, thereby identifying the biliopancreatic anatomical structures. The method combines current biliopancreatic site information, image features of the biliopancreatic anatomical structures, and position coordinates of the biliopancreatic anatomical structures to comprehensively identify and label the biliopancreatic anatomical structures, which significantly reduces difficulty of identifying the biliopancreatic anatomical structures in the biliopancreatic ultrasound images.

In some embodiments of the present disclosure, the acquisition module 1201 is specifically configured to: obtain a plurality of initial biliopancreatic ultrasound images of the biliopancreatic structure of the human body to be identified; determine an effective area in each of the initial biliopancreatic ultrasound images to obtain a plurality of the effective areas; obtain a horizontally circumscribed rectangle of each of the effective areas to obtain a plurality of the horizontally circumscribed rectangles; and respectively cut the initial biliopancreatic ultrasound images of the biliopancreatic structure to be identified along the horizontally circumscribed rectangles to obtain the biliopancreatic ultrasound images for subsequent identification.

In some embodiments of the present disclosure, the first identification module 1202 is specifically configured to: obtain a plurality of preset biliopancreatic site identification initial models; respectively train the preset biliopancreatic site identification initial models to obtain a plurality of biliopancreatic site identification models for respectively identifying different biliopancreatic sites; and identify and determine one biliopancreatic site corresponding to each of the first biliopancreatic ultrasound images of the biliopancreatic structure through the biliopancreatic site identification models. There are multiple first biliopancreatic ultrasound images, and one biliopancreatic site corresponding to each of the biliopancreatic ultrasound images is determined.

In a specific embodiment, the number of preset biliopancreatic site identification initial models is eight. The first identification module 1202 is specifically configured to: respectively train eight preset biliopancreatic site recognition initial models to obtain eight biliopancreatic site recognition models, wherein the eight biliopancreatic site identification models are used to respectively identify an abdominal aorta site, a gastric cavity and pancreatic body site, a gastric cavity and pancreatic tail site, a confluence site, a first hepatic portal site, a gastric cavity and pancreatic head site, a duodenal bulb site, and a duodenal descending part site in the biliopancreatic ultrasound images of the biliopancreatic structure.

In some embodiments of the present disclosure, the second identification module 1203 is specifically configured to: obtain a plurality of preset biliopancreatic anatomical structure identification models; and sequentially use the preset biliopancreatic anatomical structure identification models as a target biliopancreatic anatomical structure identification model to identify and determine the biliopancreatic anatomical structures in each of the first biliopancreatic ultrasound images. Images in which the biliopancreatic anatomical structures are identifiable in the first biliopancreatic ultrasound images are the second biliopancreatic ultrasound images. Images in which the biliopancreatic anatomical structures are unidentifiable in the first biliopancreatic ultrasound images are the third biliopancreatic ultrasound images.

In some embodiments, the positioning module 1204 is specifically configured to: determine a plurality of target regions in the third biliopancreatic ultrasound images, wherein each of the target regions is surrounded by a plurality of initial edge points; determine a coordinate origin in each of the third biliopancreatic ultrasound images to determine coordinates of the initial edge points of each of the target regions; determine coordinates of a center point of each of the target regions according to the coordinates of the initial edge points of each of the target regions; obtain a preset positional relationship of the biliopancreatic anatomical structures; and determine the biliopancreatic anatomical structures in the target regions according to the positional relationship and the coordinates of the center point. The biliopancreatic anatomical structures in the target regions are the biliopancreatic anatomical structures in the third biliopancreatic ultrasound images.

In some embodiments, the positioning module 1204 is specifically configured to: perform preset sparse and homogenization processing on the initial edge points to obtain a plurality of edge points; determine coordinates of the edge points according to the coordinate origin; and determine the coordinates of the center point of each of the target regions according to the coordinates of the edge points.

This disclosure further provides a server integrated with any device for identifying the biliopancreatic ultrasound images provided by the disclosure. Please refer to FIG. 13, which is a schematic structural diagram of a server according to an embodiment of the present disclosure, and details are as follows.

The server may comprise a processor 1301 with one or more processing cores, a memory 1302 with one or more computer-readable storage media, a power supply 1303, an input unit 1304, and the like. Those skilled in the art can understand that the server shown in FIG. 13 does not constitute a limitation on the server. The server may comprise more or fewer components than those shown in FIG. 13, combine some components, or have a different arrangement of components.

The processor 1301 is a control center of the server. The processor 1301 connects various parts of the server through various interfaces and lines. The processor 1301 executes various functions of the server and processes data by running or executing software programs and/or models stored in the memory 1302 and calling data stored in the memory 1302 to monitor the server. Optionally, the processor 1301 may comprise one or more processing cores. Preferably, the processor 1301 may integrate an application processor and a modem processor. The application processor mainly handles an operating system, a user interface, and application programs. The modem processor mainly handles wireless communication. It can be understood that the modem processor may not be integrated into the processor 1301.

The memory 1302 may be configured to store software programs and models. The processor 1301 executes various functional applications and data processing by executing the software programs and models stored in the memory 1302. The memory 1302 may mainly comprise a stored program area and a stored data area. The stored program area may store an operating system, an application program required for at least one function (such as a sound playback function, an image playback function, and the like), and the like. The storage data area may store data or the like created according to use of the server. In addition, the memory 1302 may comprise a high-speed random access memory, and may further comprise a non-volatile memory, for example, at least one disk memory device, a flash memory device, or other volatile solid memory device. Accordingly, the memory 1302 may further comprise a memory controller to provide the processor 1301 access to the memory 1302.

The server further comprises the power supply 1303 for powering various components. Preferably, the power supply 1303 may be logically connected to the processor 1301 through a power management system, thereby implementing functions such as charging management, discharging management, and power consumption management by using the power management system. The power supply 1303 may comprise one or more of a direct current or alternating current power supply, a re-charging system, a power failure detection circuit, a power supply converter or inverter, a power supply state indicator, and other components.

The server may further comprise the input unit 1304. The input unit 1304 is configured to receive input numerical or character information, and generate signal inputs of a keypad, a mouse, a joystick, an optical device, or a trackball related to user settings and function control.

Although not shown, the server may further comprise a display unit and the like, which will not be described in details herein. Specifically in this embodiment, the processor 1301 of the server loads executable files corresponding to processes of one or more application programs into the memory 1302 according to the following instructions, and the application programs stored in the memory 1302 are executed by the processor 1301, thereby implementing various functions:

obtaining the biliopancreatic ultrasound images of a biliopancreatic structure of a human body to be identified;
identifying and determining a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;

identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and

performing position identification on the third biliopancreatic ultrasound images to determine the biliopancreatic anatomical structures in the third biliopancreatic ultrasound images.

The present disclosure further provides a computer-readable storage medium. The storage medium may comprise a read-only memory (ROM), a random access memory (RAM), a magnetic disk, an optical disk, or the like. The storage medium stores computer programs. The computer programs are loaded by a processor to execute any method for identifying the biliopancreatic ultrasound images provided in the embodiments of the present disclosure. For example, the computer programs are loaded by the processor to perform the following steps:

obtaining the biliopancreatic ultrasound images of a biliopancreatic structure of a human body to be identified;

identifying and determining a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;

identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and

performing position identification on the third biliopancreatic ultrasound images to determine the biliopancreatic anatomical structures in the third biliopancreatic ultrasound images.

It should be noted that the method of the present disclosure is executed by an electronic device. Objects processed by the electronic device are all in form of data or information, such as time, which is essentially time information. It can be understood that if size, quantity, location, etc. are mentioned in the subsequent embodiments, they are all corresponding data so as to be processed by the electronic device, which will not be described in details herein.

The method and device for identifying the biliopancreatic ultrasound images and the server provided by the present disclosure are described in detail above. The present disclosure uses specific examples to describe principles and embodiments of the present invention. The above description of the embodiments is only for helping to understand solutions of the present disclosure and its core ideas. Furthermore, those skilled in the art may make modifications to the specific embodiments and applications according to ideas of the present invention. In conclusion, the present specification should not be construed as a limitation to the present invention.

**Claims**

1. A method for identifying a plurality of biliopancreatic ultrasound images, comprising:

obtaining the biliopancreatic ultrasound images of a biliopancreatic structure of a human body to be identified;

identifying and determining a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;

identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and

performing position identification on the third biliopancreatic ultrasound images to determine the biliopancreatic anatomical structures in the third biliopancreatic ultrasound images.

**2.** The method for identifying the biliopancreatic ultrasound images as claimed in claim 1, **characterized in that** the obtaining the biliopancreatic ultrasound images of the biliopancreatic structure of the human body to be identified comprises:

obtaining a plurality of initial biliopancreatic ultrasound images of the biliopancreatic structure of the human body to be identified;
determining an effective area in each of the initial biliopancreatic ultrasound images to obtain a plurality of the effective areas;
obtaining a horizontally circumscribed rectangle of each of the effective areas to obtain a plurality of the horizontally circumscribed rectangles; and
respectively cutting the initial biliopancreatic ultrasound images of the biliopancreatic structure to be identified along the horizontally circumscribed rectangles to obtain the biliopancreatic ultrasound images for subsequent identification.

**3.** The method for identifying the biliopancreatic ultrasound images as claimed in claim 2, **characterized in that** the identifying and determining the biliopancreatic sites corresponding to the biliopancreatic ultrasound images of the biliopancreatic structure through the preset biliopancreatic site identification model to obtain the first biliopancreatic ultrasound images comprises:

obtaining a plurality of preset biliopancreatic site identification initial models;
respectively training the preset biliopancreatic site identification initial models to obtain a plurality of biliopancreatic site identification models for respectively identifying different biliopancreatic sites; and
identifying and determining one biliopancreatic site corresponding to each of the first biliopancreatic ultrasound images of the biliopancreatic structure through the biliopancreatic site identification models, wherein there are multiple first biliopancreatic ultrasound images, and one biliopancreatic site corresponding to each of the biliopancreatic ultrasound images is determined.

**4.** The method for identifying the biliopancreatic ultrasound images as claimed in claim 3, **characterized in that** the respectively training the preset biliopancreatic site identification initial models to obtain the biliopancreatic site identification models for respectively identifying different biliopancreatic sites comprises: training a plurality of neural network (ResNet) models to obtain the biliopancreatic site identification models, wherein the biliopancreatic site identification models respectively identify different biliopancreatic sites.

**5.** The method for identifying the biliopancreatic ultrasound images as claimed in claim 3, **characterized in that** the number of the preset biliopancreatic site identification initial models is eight, and the respectively training the preset biliopancreatic site identification initial models to obtain the biliopancreatic site identification models for respectively identifying different biliopancreatic sites comprises:
respectively training the eight preset biliopancreatic site identification initial models to obtain eight biliopancreatic site identification models, wherein the eight biliopancreatic site identification models are used to respectively identify an abdominal aorta site, a gastric cavity and pancreatic body site, a gastric cavity and pancreatic tail site, a confluence site, a first hepatic portal site, a gastric cavity and pancreatic head site, a duodenal bulb site, and a duodenal descending part site in the biliopancreatic ultrasound images of the biliopancreatic structure.

**6.** The method for identifying the biliopancreatic ultrasound images as claimed in claim 2, **characterized in that** the determining the effective area in each of the initial biliopancreatic ultrasound images to obtain the plurality of the effective areas comprises:

training an UNet++ image neural network model; and
identifying the effective area in each of the initial biliopancreatic ultrasound images through the trained UNet++ image neural network model to obtain the plurality of the effective areas.

**7.** The method for identifying the biliopancreatic ultrasound images as claimed in claim 1, **characterized in that** the identifying a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images comprises:

obtaining a plurality of preset biliopancreatic anatomical structure identification models; and
sequentially using the preset biliopancreatic anatomical structure identification models as a target biliopancreatic anatomical structure identification model to identify and determine the biliopancreatic anatomical structures in each of the first biliopancreatic ultrasound images, wherein images in which the biliopancreatic anatomical structures are identifiable in the first biliopancreatic ultrasound images are the second biliopancreatic ultrasound images, and images in which the biliopancreatic anatomical structures are unidentifiable in the first biliopancreatic ultrasound images are the third biliopancreatic ultrasound images.

8. The method for identifying the biliopancreatic ultrasound images as claimed in claim 7, **characterized in that** the performing position identification on the third biliopancreatic ultrasound images to determine the biliopancreatic anatomical structures in the third biliopancreatic ultrasound images comprises:

   determining a plurality of target regions in the third biliopancreatic ultrasound images, wherein each of the target regions is surrounded by a plurality of initial edge points;
   determining a coordinate origin in each of the third biliopancreatic ultrasound images to determine coordinates of the initial edge points of each of the target regions;
   determining coordinates of a center point of each of the target regions according to the coordinates of the initial edge points of each of the target regions;
   obtaining a preset positional relationship of the biliopancreatic anatomical structures; and
   determining the biliopancreatic anatomical structures in the target regions according to the positional relationship and the coordinates of the center point, wherein the biliopancreatic anatomical structures in the target regions are the biliopancreatic anatomical structures in the third biliopancreatic ultrasound images.

9. The method for identifying the biliopancreatic ultrasound images as claimed in claim 8, **characterized in that** the determining the coordinates of the center point of each of the target regions according to the coordinates of the initial edge points of each of the target regions comprises:

   performing preset sparse and homogenization processing on the initial edge points to obtain a plurality of edge points;
   determining coordinates of the edge points according to the coordinate origin; and
   determining the coordinates of the center point of each of the target regions according to the coordinates of the edge points.

10. The method for identifying the biliopancreatic ultrasound images as claimed in claim 9, **characterized in that** the performing the preset sparse and homogenization processing on the initial edge points to obtain the edge points comprises:

traversing the initial edge points in a preset order;
discarding the initial edge points whose distance between any two adjacent initial edge points is less than 10 pixels; and
inserting a new initial edge point between any two adjacent initial edge points with a distance greater than 10 pixels to obtain the edge points.

11. The method for identifying the biliopancreatic ultrasound images as claimed in claim 9, **characterized in that** the determining the coordinates of the center point of each of the target regions according to the coordinates of the edge points comprises:
averaging the coordinates of the edge points of each of the target regions to obtain a mean value as the coordinates of the center point of each of the target regions.

12. The method for identifying the biliopancreatic ultrasound images as claimed in claim 7, **characterized in that** the anatomical structures are divided into seven categories, and the obtaining the preset biliopancreatic anatomical structure identification models comprises:
obtaining seven preset biliopancreatic anatomical structure identification models.

13. A device for identifying a plurality of biliopancreatic ultrasound images, comprising:

   an acquisition module configured to obtain the biliopancreatic ultrasound images of a biliopancreatic structure of a human body to be identified;
   a first identification module configured to identify and determine a plurality of biliopancreatic sites corresponding to the biliopancreatic ultrasound images of the biliopancreatic structure through a preset biliopancreatic site identification model to obtain a plurality of first biliopancreatic ultrasound images, wherein one biliopancreatic site corresponding to each of the first biliopancreatic ultrasound images is determined;
   a second identification module configured to identify a plurality of biliopancreatic anatomical structures in the first biliopancreatic ultrasound images through a preset biliopancreatic anatomical structure identification model to determine a plurality of second biliopancreatic ultrasound images in which the biliopancreatic anatomical structures are identifiable in the first biliopancreatic ultrasound images and a plurality of third biliopancreatic ultrasound images in which the biliopancreatic anatomical structures are unidentifiable in the first biliopancreatic ultrasound images; and
   a positioning module configured to perform position identification on the third biliopancreatic ultrasound images to determine the biliopancreatic anatomical structures in the third biliopancreatic ultrasound images.

14. The device for identifying the biliopancreatic ultrasound images as claimed in claim 13, **characterized in that** the acquisition module is specifically configured to:

obtain a plurality of initial biliopancreatic ultrasound images of the biliopancreatic structure of the human body to be identified;
determine an effective area in each of the initial biliopancreatic ultrasound images to obtain a plurality of the effective areas;
obtain a horizontally circumscribed rectangle of each of the effective areas to obtain a plurality of the horizontally circumscribed rectangles; and
respectively cut the initial biliopancreatic ultrasound images of the biliopancreatic structure to be identified along the horizontally circumscribed rectangles to obtain the biliopancreatic ultrasound images for subsequent identification.

15. The device for identifying the biliopancreatic ultrasound images as claimed in claim 13, **characterized in that** the first identification module is specifically configured to:

obtain a plurality of preset biliopancreatic site identification initial models;
respectively train the preset biliopancreatic site identification initial models to obtain a plurality of biliopancreatic site identification models for respectively identifying different biliopancreatic sites; and
identify and determine one biliopancreatic site corresponding to each of the first biliopancreatic ultrasound images of the biliopancreatic structure through the biliopancreatic site identification models, wherein there are multiple first biliopancreatic ultrasound images, and one biliopancreatic site corresponding to each of the biliopancreatic ultrasound images is determined.

16. The device for identifying the biliopancreatic ultrasound images as claimed in claim 15, **characterized in that** the number of the preset biliopancreatic site identification initial models is eight, and the first identification module is specifically configured to:
respectively train eight preset biliopancreatic site identification initial models to obtain eight biliopancreatic site identification models, wherein the eight biliopancreatic site identification models are used to respectively identify an abdominal aorta site, a gastric cavity and pancreatic body site, a gastric cavity and pancreatic tail site, a confluence site, a first hepatic portal site, a gastric cavity and pancreatic head site, a duodenal bulb site, and a duodenal descending part site in the biliopancreatic ultrasound images of the biliopancreatic structure.

17. The device for identifying the biliopancreatic ultrasound images as claimed in claim 13, **characterized in that** the second identification module is specifically configured to:

obtain a plurality of preset biliopancreatic anatomical structure identification models; and
sequentially use the preset biliopancreatic anatomical structure identification models as a target biliopancreatic anatomical structure identification model to identify and determine the biliopancreatic anatomical structures in each of the first biliopancreatic ultrasound images, wherein images in which the biliopancreatic anatomical structures are identifiable in the first biliopancreatic ultrasound images are the second biliopancreatic ultrasound images, and images in which the biliopancreatic anatomical structures are unidentifiable in the first biliopancreatic ultrasound images are the third biliopancreatic ultrasound images.

18. The device for identifying the biliopancreatic ultrasound images as claimed in claim 17, **characterized in that** the positioning module is specifically configured to:

determine a plurality of target regions in the third biliopancreatic ultrasound images, wherein each of the target regions is surrounded by a plurality of initial edge points;
determine a coordinate origin in each of the third biliopancreatic ultrasound images to determine coordinates of the initial edge points of each of the target regions;
determine coordinates of a center point of each of the target regions according to the coordinates of the initial edge points of each of the target regions;
obtain a preset positional relationship of the biliopancreatic anatomical structures; and
determine the biliopancreatic anatomical structures in the target regions according to the positional relationship and the coordinates of the center point, wherein the biliopancreatic anatomical structures in the target regions are the biliopancreatic anatomical structures in the third biliopancreatic ultrasound images.

19. The device for identifying the biliopancreatic ultrasound images as claimed in claim 18, **characterized in that** the positioning module is specifically configured to:

perform preset sparse and homogenization processing on the initial edge points to obtain a plurality of edge points;
determine coordinates of the edge points according to the coordinate origin; and
determine the coordinates of the center point of each of the target regions according to the coordinates of the edge points.

**20.** A server, comprising:

one or more processors;
a memory; and
one or more application programs stored in the memory and configured to be executed by the processors to implement the method for identifying the biliopancreatic ultrasound images as claimed in claim 1.

detection terminal

101

image data

server

memory

200

300

user terminal

102

## FIG. 1

obtaining the biliopancreatic ultrasound images of
a biliopancreatic structure of a human body to be identified

21

identifying and determining a plurality of biliopancreatic sites
corresponding to the biliopancreatic ultrasound images of the
biliopancreatic structure through a preset biliopancreatic site identification
model to obtain a plurality of first biliopancreatic ultrasound images

22

identifying a plurality of biliopancreatic anatomical structures in the first
biliopancreatic ultrasound images through a preset biliopancreatic
anatomical structure identification model to determine a plurality of second
biliopancreatic ultrasound images in which the biliopancreatic anatomical
structures are identifiable in the first biliopancreatic ultrasound images
and a plurality of third biliopancreatic ultrasound images in which
the biliopancreatic anatomical structures are unidentifiable
in the first biliopancreatic ultrasound image

23

performing position identification on the third biliopancreatic ultrasound
images to determine the biliopancreatic anatomical structures
in the third biliopancreatic ultrasound images

24

## FIG. 2

| Site number | Site name | Anatomical structure 1 | Anatomical structure 2 | Anatomical structure 3 | Anatomical structure 4 | Anatomical structure 4 |
|---|---|---|---|---|---|---|
| 1 | abdominal aorta site | abdominal aorta | celiac trunk | superior mesenteric artery | | |
| 2 | gastric cavity and pancreatic body site | splenic artery and vein | pancreatic body | | | |
| 3 | gastric cavity and pancreatic tail site | pancreatic tail | splenic artery and vein | kidney | spleen | |
| 4 | confluence site | superior mesenteric vein | portal vein | splenic vein | pancreatic neck | bile duct |
| 5 | first hepatic portal site | liver | portal vein | bile duct | | |
| 6 | gastric cavity and pancreatic head site | pancreatic duct | portal vein | bile duct | pancreatic head | |
| 7 | duodenal bulb site | bile duct | portal vein | pancreas | | |
| 8 | duodenal descending part site | ampulla | bile duct | pancreatic duct | superior mesenteric artery and vein | intestinal lumen |

**FIG. 3**

| Category | Anatomical structures | | |
|---|---|---|---|
| 1 | abdominal aorta | superior mesenteric artery | splenic artery and vein |
| | celiac trunk | superior mesenteric vein | portal vein |
| | bile duct | superior mesenteric artery and vein | splenic vein |
| | pancreatic duct | | |
| 2 | pancreatic body | pancreatic tail | pancreatic neck |
| | pancreatic head | pancreas | |
| 3 | kidney | | |
| 4 | spleen | | |
| 5 | liver | | |
| 6 | ampulla | | |
| 7 | intestinal lumen | | |

# FIG. 4

| Site number | Site name | Anatomical structure 1 | Anatomical structure 2 | Anatomical structure 3 | Anatomical structure 4 | Anatomical structure 4 |
|---|---|---|---|---|---|---|
| 1 | abdominal aorta site | | | | | |
| 2 | gastric cavity and pancreatic body site | splenic artery and vein | pancreatic body | | | |
| 3 | gastric cavity and pancreatic tail site | pancreatic tail | splenic artery and vein | kidney | spleen | |
| 4 | confluence site | | | | pancreatic neck | |
| 5 | first hepatic portal site | liver | | | | |
| 6 | gastric cavity and pancreatic head site | | | | pancreatic head | |
| 7 | duodenal bulb site | | | pancreas | | |
| 8 | duodenal descending part site | ampulla | | | | intestinal lumen |

## FIG. 5

EP 4 383 098 A1

| Site number | Site name | Anatomical structure 1 | Anatomical structure 2 | Anatomical structure 3 | Anatomical structure 4 | Anatomical structure 4 |
|---|---|---|---|---|---|---|
| 1 | abdominal aorta site | abdominal aorta | celiac trunk | superior mesenteric artery | | |
| 2 | gastric cavity and pancreatic body site | | | | | |
| 3 | gastric cavity and pancreatic tail site | | | | | |
| 4 | confluence site | superior mesenteric vein | portal vein | splenic vein | | bile duct |
| 5 | first hepatic portal site | | portal vein | bile duct | | |
| 6 | gastric cavity and pancreatic head site | pancreatic duct | portal vein | bile duct | | |
| 7 | duodenal bulb site | bile duct | portal vein | | | |
| 8 | duodenal descending part site | | bile duct | pancreatic duct | superior mesenteric artery and vein | |

# FIG. 6

23

obtaining a plurality of initial biliopancreatic ultrasound images of the biliopancreatic structure of the human body to be identified — 71

determining an effective area in each of the initial biliopancreatic ultrasound images to obtain a plurality of the effective areas — 72

obtaining a horizontally circumscribed rectangle of each of the effective areas to obtain a plurality of the horizontally circumscribed rectangles — 73

respectively cutting the initial biliopancreatic ultrasound images of the biliopancreatic structure to be identified along the horizontally circumscribed rectangles to obtain the biliopancreatic ultrasound images for subsequent identification — 74

**FIG. 7**

obtaining a plurality of preset biliopancreatic site identification initial models — 81

respectively training the preset biliopancreatic site identification initial models to obtain a plurality of biliopancreatic site identification models for respectively identifying different biliopancreatic sites — 82

identifying and determining one biliopancreatic site corresponding to each of the first biliopancreatic ultrasound images of the biliopancreatic structure through the biliopancreatic site identification models — 83

**FIG. 8**

determining a plurality of target regions in the third biliopancreatic ultrasound images — 91

determining a coordinate origin in each of the third biliopancreatic ultrasound images to determine coordinates of the initial edge points of each of the target regions — 92

determining coordinates of a center point of each of the target regions according to the coordinates of the initial edge points of each of the target regions — 93

obtaining a preset positional relationship of the biliopancreatic anatomical structures — 94

determining the biliopancreatic anatomical structures in the target regions according to the positional relationship and the coordinates of the center point — 95

## FIG. 9

| Site No. | Site name | Category 1 identification | Category 2 identification | Category 3 identification | Category 4 identification | Category 5 identification | Category 6 identification | Category 7 identification |
|---|---|---|---|---|---|---|---|---|
| 1 | abdominal aorta site | 3 | None | None | None | None | None | None |
| 2 | gastric cavity and pancreatic body site | 1 | 1 | None | None | None | None | None |
| 3 | gastric cavity and pancreatic tail site | 1 | 1 | 1 | 1 | None | None | None |
| 4 | confluence site | 4 | 1 | None | None | None | None | None |
| 5 | first hepatic portal site | 2 | None | None | None | 1 | None | None |
| 6 | gastric cavity and pancreatic head site | 3 | 1 | None | None | None | None | None |
| 7 | duodenal bulb site | 2 | 1 | None | None | None | None | None |
| 8 | duodenal descending part site | 3 | None | None | None | None | 1 | 1 |

## FIG. 10

**FIG. 11**

**FIG. 12**

**FIG. 13**

## INTERNATIONAL SEARCH REPORT

| International application No. |
| --- |
| **PCT/CN2021/143710** |

**A. CLASSIFICATION OF SUBJECT MATTER**

G06K 9/62(2022.01)i; G06T 7/00(2017.01)i; A61B 8/08(2006.01)i

According to International Patent Classification (IPC) or to both national classification and IPC

**B. FIELDS SEARCHED**

Minimum documentation searched (classification system followed by classification symbols)

G06K; G06T; A61B

Documentation searched other than minimum documentation to the extent that such documents are included in the fields searched

Electronic data base consulted during the international search (name of data base and, where practicable, search terms used)

CNABS, CNTXT, CNKI, DWPI, VEN, USTXT, EPTXT, WOTXT, IEEE: 解剖, 胰腺, 胆胰, 超声, 检查, 站点, 结构, 识别, 分类, 深度学习, 神经网络, CNN, 不可识别, 不合格, 无效, 失败, 坐标, 位置, 区域, endoscop??, anatom+, dissect+, ultra+, recogni+, detect+, classif+, deep learn???, coordinat+, site, area, ResNet

**C. DOCUMENTS CONSIDERED TO BE RELEVANT**

| Category* | Citation of document, with indication, where appropriate, of the relevant passages | Relevant to claim No. |
| --- | --- | --- |
| PX | CN 113344926 A (WUHAN CHUJINGLING MEDICAL TECHNOLOGY CO., LTD.) 03 September 2021 (2021-09-03) description, paragraphs 23-107 | 1-20 |
| Y | CN 109614995 A (RENMIN HOSPITAL OF WUHAN UNIVERSITY, HUBEI PROVINCIAL RENMIN HOSPITAL) 12 April 2019 (2019-04-12) description, paragraphs 20-41 | 1-20 |
| Y | CN 111415564 A (WUHAN UNIVERSITY) 14 July 2020 (2020-07-14) description, paragraphs 31-36 | 1-20 |
| Y | US 2020245960 A1 (KLOX KLOX TECHNOLOGIES IN) 06 August 2020 (2020-08-06) description, paragraphs 20-135 | 8-11, 18-19 |
| A | CN 113012140 A (WUHAN CHUJINGLING MEDICAL TECHNOLOGY CO., LTD.) 22 June 2021 (2021-06-22) entire document | 1-20 |
| A | CN 111582215 A (UNION HOSPITAL, TONGJI MEDICAL COLLAGE, HUAZHONG UNIVERSITY OF SCIENCE AND TECHNOLOGY) 25 August 2020 (2020-08-25) entire document | 1-20 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 March 2022** | **20 April 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2021/143710**

| Patent document cited in search report | | | Publication date (day/month/year) | Patent family member(s) | | | Publication date (day/month/year) |
|---|---|---|---|---|---|---|---|
| CN | 113344926 | A | 03 September 2021 | CN | 113344926 | B | 02 November 2021 |
| CN | 109614995 | A | 12 April 2019 | None | | | |
| CN | 111415564 | A | 14 July 2020 | None | | | |
| US | 2020245960 | A1 | 06 August 2020 | EP | 3909014 | A1 | 17 November 2021 |
| | | | | CN | 113272859 | A | 17 August 2021 |
| | | | | AU | 2020206584 | A1 | 17 June 2021 |
| | | | | WO | 2020144134 | A1 | 16 July 2020 |
| | | | | TW | 202040518 | A | 01 November 2020 |
| | | | | CA | 3122540 | A1 | 16 July 2020 |
| | | | | JP | 2022516316 | A | 25 February 2022 |
| CN | 113012140 | A | 22 June 2021 | None | | | |
| CN | 111582215 | A | 25 August 2020 | None | | | |

Form PCT/ISA/210 (patent family annex) (January 2015)